# EUROPÄISCHE PATENTANMELDUNG

(11) **EP 2 039 305 A1**
(43) Veröffentlichungstag der Anmeldung: **25.03.2009**
(21) Anmeldenummer: 08164654.9
(22) Anmeldetag: 18.09.2008
(51) Int. Cl.: A61B 17/15

(54) **Bearbeitungslehre zur Herstellung einer Resektionsfläche an einem Gelenkknochen**

(30) Priorität: 18.09.2007 DE 202007013156 U
(71) Anmelder: Zrinski AG, 78573 Wurmlingen (DE)
(72) Erfinder: Lindner, Nicola, 78573, Wurmlingen (DE); Eckhof, Stephan, 78604, Weilheim-Rietheim (DE); Feldhaus, Thomas, 78532, Tuttlingen (DE)
(74) Vertreter: Muri, Peter

(57) **Zusammenfassung**

Die Erfindung bezieht sich auf eine Bearbeitungslehre zur Herstellung einer Resektionsfläche an einem Knochen zum Anpassen eines Implantats, wobei die Bearbeitungslehre zumindest ein Auflage- und Fixierelement zum Auflegen und Befestigen an dem Knochen und eine Anlagefläche zum Führen eines Werkzeugs umfasst.

Erfindungsgemäss ist vorgesehen, dass die Bearbeitungslehre (S) ein Anschlusselement (3) umfasst, das geeignet ist, eine formschlüssige Verbindung zu einem Adapterelement bereitzustellen, das wiederum mittelbar oder unmittelbar an ein Werkzeug zur Herstellung einer Aushöhlung (45) in dem Knochen geeignet ist.

## Beschreibung

Die Erfindung bezieht sich auf eine Bearbeitungslehre zur Herstellung einer Resektionsfläche an einem Knochen zum Anpassen eines Implantats, wobei die Bearbeitungslehre zumindest ein Auflage- und Fixierelement zum Auflegen und Befestigen an dem Knochen und eine Anlagefläche zum Führen eines Werkzeugs umfasst.

### Stand der Technik

Zur Herstellung von Resektionsflächen, beispielsweise an Fingerknochen, wird in der Regel bei Operationsverfahren sehr oft freihändig gearbeitet. Dies bedeutet, dass für die Durchführung der Operationstechnik, nämlich der Herstellung einer Resektionsfläche zur Aufnahme von Implantaten viel Erfahrung notwendig ist. Der Operateur meisselt oder bohrt entsprechende Aufnahmen im Röhrenknochen einer proximalen Phalanx vor, die später für die Aufnahme eines Schaftes eines Implantats geeignet ist. Mit einer oszillierenden Säge werden die entsprechenden Resektionsflächen, vorzugsweise zunächst senkrecht zur Längserstreckung der proximalen Phalanx und dann in einem Winkel von 60 Grad zur Längserstreckung der proximalen Phalanx hergestellt.

Um solche Operationstechniken zu verbessern und auch die Genauigkeit der Relation der Resektionsfläche zum Implantat zu erhöhen, sind unterschiedliche Vorschläge aus dem Stand der Technik bekannt. Insbesondere Bearbeitungslehren dienen dazu, Sägeschnitte durchzuführen, die zu einer bestimmten Bezugsebene ausgeführt werden. Bearbeitungslehren der vorstehenden Art weisen Auflageelemente auf, an denen das Sägeblatt angelegt und geführt werden kann. Fixierelemente dienen dazu, die Bearbeitungslehre an dem zu bearbeitenden Knochen zu befestigen. So kann beispielsweise eine Bearbeitungslehre auf der proximalen Phalanx aufgelegt und dort befestigt werden, so dass mit einer oszillierenden Säge an einer Anlagefläche die entsprechende Resektionsfläche hergestellt wird.

Bearbeitungslehren sind aber auch aus benachbarten Gebieten von medizinischen Operationstechniken bekannt.

So ist beispielsweise aus der DE 19516294 A (MATTHIAS POTHMANN) 04.05.1995 eine Bearbeitungslehre bekannt. Die Bearbeitungslehre besteht aus einer Vorrichtung umfassend eine Sägeblattführung, die als Führungsschiene ausgebildet ist, die über ein Verbindungsstück an einem Kugelgelenk gelagert ist und dadurch einerseits zwischen zwei Spannbacken verschwenkbar angeordnet ist. Die Winkelpositionseinstellung dient dazu, die Lage des Sägeblattes in Bezug auf den Knochen, der mechanisch bearbeitet wird, auszurichten.

Die Einstellung ist jedoch sehr komplex und umfasst sehr viele Bauteile.
Zudem ist sie nur im Kniegelenksbereich anwendbar.

So ist auch aus der US 5911724 A (ULRICH WEHRLI) 21.05.1996 eine sehr kompakt bauende, aber aus vielen Bauelementen bestehende Vorrichtung bekannt. Mittels dieser Vorrichtung kann die Sägeblattführung sehr genau eingestellt werden. Aber auch diese eignet sich nicht für den Finger- und Fussgelenksbereich.

Aus der EP 1476084 B (KARL STORZ GMBH & CO KG) 28.01.2003 ist eine Bearbeitungslehre für medizinische Zwecke dargestellt, die eine genaue Führung des Sägeblatts zulässt. Der Aufbau der Vorrichtung ist kompakt und besteht aus wenigen Bauteilen. Aufgrund der Grösse der Spanneinrichtung ist es jedoch auch nicht möglich, für Fingergelenks- oder Fuss- und Zehenbereich einzusetzen.

Aus der DE 4423717 C (ESCA MEDICAL GMBH & CO) 08.07.1994 ist eine Vorrichtung zur Festlegung von Resektionsflächen an Femur und an der Tibia zur Vorbereitung einer Implantation an der Kniegelenksprothese bekannt. Zur lagegerechten Ausrichtung der Resektionsfläche sind Spannvorrichtungen vorgesehen, die sowohl im Bereich der Femur als auch in der Tibia eingesetzt werden. Die Lage der Vorrichtung ist reproduzierbar positionierbar und stellt somit eine femorale Bearbeitungslehre dar. Sie ist vorzugsweise, da anwendbar im Bereich des Knies, auch drehgelenkig beweglich. Auch diese Vorrichtung dient dazu, ausschliesslich im Hüft- oder Kniegelenksbereich Anwendung zu finden. Im Fingergelenksbereich oder Fuss- beziehungsweise Zehenbereich ist diese zu kompakt und weist mechanisch zu viele Bauteile auf, so dass sie nicht auf die entsprechende Anwendung übertragbar ist.

### Nachteile des Standes der Technik

Wie bereits zuvor erwähnt, ist ein wesentlicher Nachteil des Standes der Technik der, dass für die Durchführung von Operationstechniken zur Herstellung von Resektionsflächen, die dazu geeignet sind, Implantaten entsprechende Anlageflächen zu verschaffen, nur mit viel Erfahrungsschatz herstellbar sind, da vorwiegend freihändig gearbeitet wird. Dies bedeutet, dass nur in seltenen Fällen Bearbeitungslehren und Bearbeitungshilfen vorgesehen sind, die eine Passgenauigkeit gewährleisten.

Die Bearbeitungslehren, die bereits zur Herstellung von Resektionsflächen, insbesondere bei Fingerknochen (proximale Phalanx) bekannt sind, weisen den Nachteil auf, dass keine Relation der Resektionsfläche zu einer Aushöhlung, die geeignet ist, einen Schaft des Implantats aufzunehmen, hergestellt ist. Dies bedeutet, dass trotz Bereitstellung einer Bearbeitungslehre zwar die Resektionsflächen zur Längserstreckung einer proximalen Phalanx lagegerecht hergestellt werden können, jedoch die Relation zur Aushöhlung fehlt. Dies bedeutet, da die Aushöhlung separat erstellt wird, ein Implantat möglicherweise auch schief sitzen kann, so dass die Funktion des Implantates an sich nicht oder zumindest nicht im vollem Umfang gewährleistet werden kann.

Ein weiterer wesentlicher Nachteil ist, dass durch entweder freihändige Arbeit, aber auch mit Zuhilfenahme der beschriebenen Bearbeitungslehre, nach Herstellung einer Aushöhlung zur Aufnahme eines Schaftes des zu implantierenden Implantats, die Resektionsflächen zur Längserstreckung der proximalen Phalanx nicht korrekt platziert sind, so dass es überhaupt nicht zur Anlage des Implantats an der Resektionsfläche kommt.

Eine funktionsgerechte Benutzung des Implantates ist damit nicht mehr möglich.

Da jedoch ein einmal zuviel abgetrenntes Knochenstück nicht mehr angesetzt werden kann, entstehen Spannungen im Bereich des implantierten Implantats, die letztendlich dazu führen, dass das Ziel, eine Wiederherstellung des Gelenkes nicht mehr weiterverfolgt werden kann und es konsequenterweise entsprechend versteift wird.

### Aufgabe der Erfindung

Daher ist es Aufgabe der Erfindung, eine Bearbeitungslehre bereitzustellen, deren herzustellenden Resektionsflächen einen Bezug zu einer in einem Knochen vorbereiteten Aushöhlung zur Aufnahme eines Schaftes eines Implantats bereitstellen.

### Lösung der Aufgabe

Die Lösung der Aufgabe wird durch den kennzeichnenden Teil von Anspruch 1 gekennzeichnet.

### Vorteile der Erfindung

Einer der wesentlichen Vorteile der Erfindung liegt darin, dass der Sägeschnitt zur Herstellung der Resektionsfläche sich an der Lage und der Tiefe der Aushöhlung zur Aufnahme des Implantatschaftes orientiert. Dadurch wird erreicht, dass der Schaft des eingesetzten Implantats korrekt von der Aushöhlung aufgenommen wird und gleichzeitig auch die Innenseite des Implantatkopfes an der Resektionsfläche zur Anlage kommt.

Die lage- und sachgerechte Funktionsaufnahme des Implantats an dem bearbeiteten Gelenkknochen ist unabhängig von der lagegerechten Ausrichtung der Aushöhlung. Unabhängig davon, ob diese nun achsparallel, beispielsweise zu einer proximalen Phalanx ausgerichtet oder aber schräg ausgebildet ist, wird immer eine Anlage eines Implantats an der Resektionsfläche erreicht. Somit sind die Achsen des Implantatschaftes und die der Resektionsfläche immer in korrekter Relation.

Ein weiterer wesentlicher Vorteil der Erfindung liegt darin, dass bei der Herstellung der Resektionsfläche keine Metallspäne entstehen. Dies ist darin begründet, dass Bearbeitungslehre beziehungsweise Sägelehre und der Bereich der Auflage und Fixierung getrennt voneinander angeordnet sind. So wird vermieden, dass Fremdkörper während der Operation in das umliegende Gewebe eindringt und so möglicherweise zu Entzündungen führen.

Die Verbindung von der die Aushöhlung bearbeitende Raspel in Bezug auf die Positionierung der Lehre zur Herstellung der Resektionsfläche ist der wesentliche Vorteil der Erfindung. Sie wird dadurch gelöst, dass eine Bearbeitungslehre bereitgestellt wird, die neben einem Aufnahme- und Fixierelement auch eine Anlagefläche zum Führen eines Werkzeugs, beispielsweise eines Sägeblattes umfasst. Zudem ist wesentlich kennzeichnend für die Bearbeitungslehre, dass mindestens ein Aufnahmebereich vorhanden ist, der zur Aufnahme von Adapterelementen dient. Eines der Adapterelemente stellt insbesondere eine formschlüssige Verbindung zwischen der in die Aushöhlung eingeführte Raspel und der Bearbeitungslehre dar, damit die zuvor beschriebene Relation hergestellt werden kann.

Zusätzlich weist diese Bearbeitungslehre vorteilhafterweise Begrenzungselemente im Bereich der Anlagefläche auf. Somit wird vorteilhafterweise vermieden, dass durch das Einsetzen eines Werkzeuges umliegendes Gewebe beschädigt werden kann.

Die Bearbeitungslehre selbst weist im Bereich des Auflage- und Fixierelements Bohrungen auf, die zur Aufnahme von Befestigungsmitteln dienen. Hier können beispielsweise Kirschner-Drähte vorgesehen werden, die in den Knochen eingebohrt werden, um dann mit der Bearbeitungslehre durch beispielsweise Verbiegen oder Verdrehen der Enden oder ähnlichem fixiert werden. Alternative Befestigungsmittel können ebenfalls vorgesehen sein. Ziel ist es, eine lagegerechte und ortsfeste Fixierung der Bearbeitungslehre auf einem Knochen zu erreichen.

Vorteilhafterweise können über den ein und denselben Aufnahmebereich auch weitere Adapterelemente angefügt werden. Diese Adapterelemente dienen insbesondere dazu, die beispielsweise senkrecht zur Längserstreckung des Knochens hergestellte Resektionsfläche weiterzubearbeiten. So können beispielsweise schräge Flächen in definierten Winkeln angebracht werden, wobei die Adapterelemente entsprechende Anlageflächen vorsehen, an denen das Werkzeug, beispielsweise ein Sägeblatt, entlang geführt werden kann.

Der erfinderische Gedanke, eine Relation zwischen der Achse der Aushöhlung und den Resektionsflächen durch entsprechende Bereitstel lung einer Bearbeitungslehre, die mit einem für die Aushöhlung bestimmten Werkzeug hergestellt werden kann, ist nicht nur auf Fingerknochen beschränkt. Vielmehr ist die erfinderische Bearbeitungslehre und auch das Verfahren überall in den Bereichen anzuwenden, in denen insbesondere Gelenkknochen durch Implantate zu ersetzen sind. So können beispielsweise solche Verfahren im Handgelenksbereich, Zehenbereich aber auch bei sonstigen Gelenkknochen verwendet werden.

Das erfinderische Verfahren stützt sich somit vorteilhafterweise auf die Verfahrensschritte, dass zunächst mittels einem definierten Werkzeug eine Aushöhlung in dem Knochen bearbeitet wird. Das Werkzeug, das in der Aushöhlung noch platziert ist, weist zusätzliche Adapterelemente auf, auf die dann die entsprechende Bearbeitungslehre fixierbar ist. Durch Fixieren der Bearbeitungslehre ist die Relation der über die Bearbeitungslehre herzustellenden Resektionsfläche zur hergestellten Aushöhlung bestimmt.

Weitere vorteilhafte Ausgestaltungen gehen aus den nachfolgenden Beschreibungen, den Zeichnungen sowie den Ansprüchen hervor.

### Zeichnungen

**Es zeigen:**

Fig. 1 eine perspektivische Ansicht auf die erfindungsgemässe Bearbeitungslehre in Schrägansicht;

Fig. 2 eine weitere perspektivische Ansicht auf die Bearbeitungslehre gemäss Figur 1;

Fig. 3 eine Seitenansicht auf die Bearbeitungslehre gemäss Figur 1;

Fig. 4 eine Draufsicht auf die Bearbeitungslehre gemäss Figur 1;

Fig. 5 eine perspektivische Ansicht auf ein Ausrichtelement, adaptierbar an die Bearbeitungslehre gemäss den Figuren 1 bis 4;

Fig. 6 eine perspektivische Ansicht auf ein erstes Adapterelement, nämlich eine Fräslehre zum Anbringen an die Bearbeitungslehre gemäss Figur 1;

Fig. 7 eine perspektivische Ansicht auf ein weiteres Adapterelement, nämlich eine Bearbeitungslehre mit schräger Anlagefläche;

Fig. 8 A-H eine schematische Darstellung des erfindungsgemässen Verfahrens in Einzelschritten zur Herstellung einer Resektionsfläche an einem Gelenk eines Knochens.

### Beschreibung eines Ausführungsbeispiels

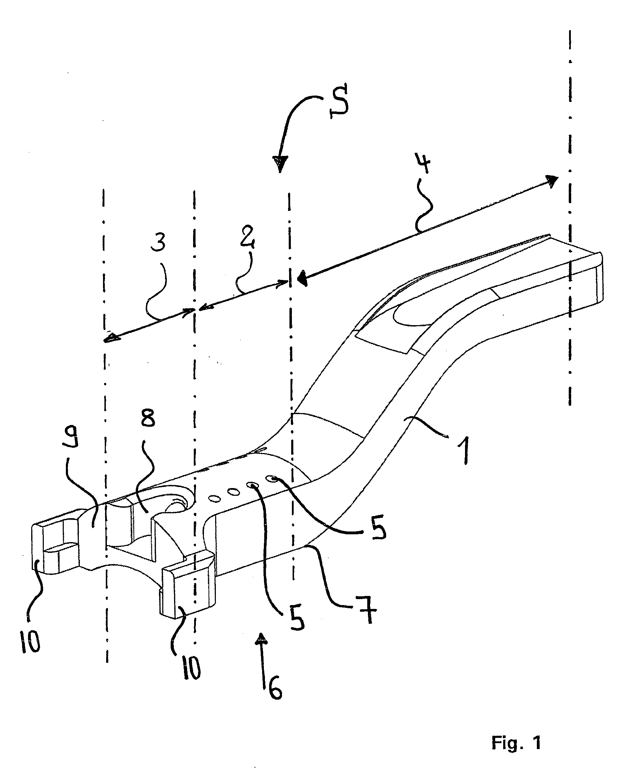

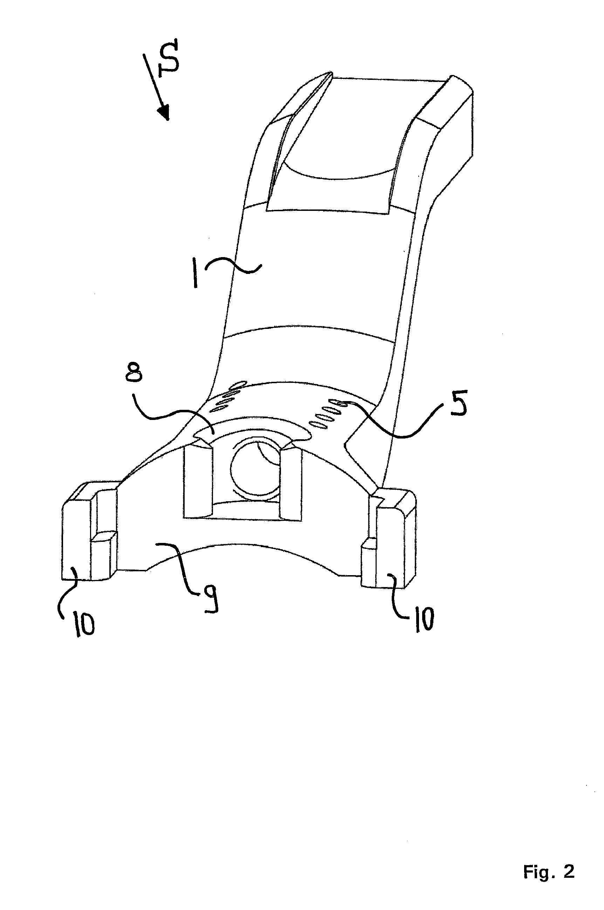

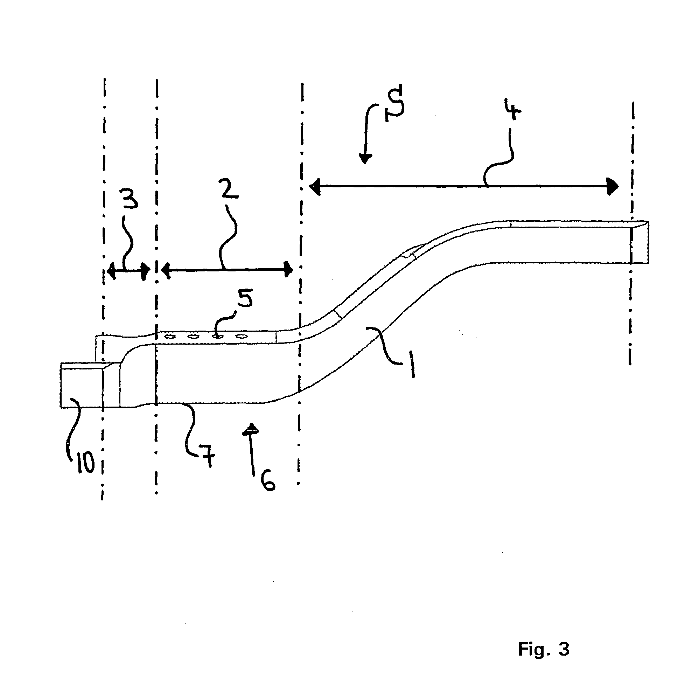

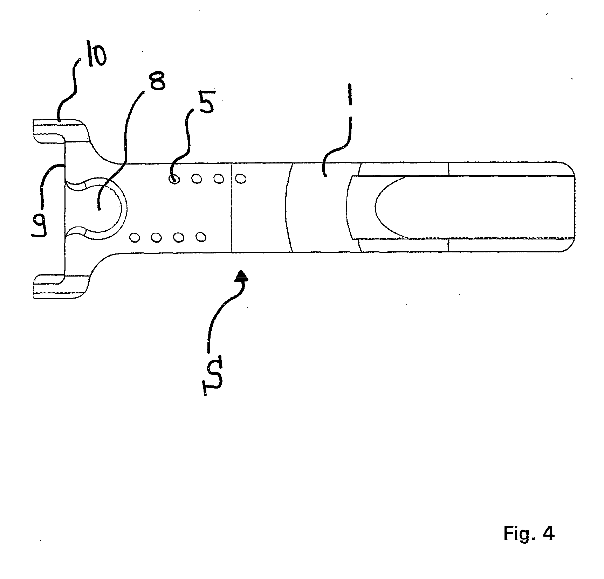

In den Figuren 1 bis 4 ist die erfindungsgemässe Bearbeitungslehre S dargestellt. Sie umfasst einen Grundkörper 1, an den unterschiedliche Adapterelemente, die nachfolgend näher beschrieben werden, adaptierbar sind.

Der Grundkörper 1 der Bearbeitungslehre S teilt sich im Wesentlichen in drei Abschnitte auf. Ein erster Abschnitt ist ein zentral angelegtes Auflage- und Fixierelement 2, das geeignet ist auf einem Knochen aufzuliegen und an diesem befestigt zu werden. Ein solcher Knochen kann beispielsweise ein Fingerknochen sein. Hiervon aus erstreckt sich zur einen Seite ein Anschlusselement 3, dem die Aufgabe zugewiesen ist, die zuvor erwähnten Adapterelemente zumindest formschlüssig aufzunehmen. Auf der gegenüberliegenden Seite erstreckt sich ein Funktionselement 4, das insbesondere dazu dient, mit den an dem Anschlusselement 3 angefügten Adapterelementen zusammenzuwirken und eine kraftschlüssige Verbindung einzugehen. Auch hierauf wird nachgehend noch näher eingegangen.

Der Grundkörper 1 der Bearbeitungslehre S weist vorzugsweise ein rechteckigen Querschnitt auf und ist derart dimensioniert, dass er auf die Auflage eines Knochens geeignet ist. Daher weist er auf seiner Unterseite 6, nämlich die Seite, die zu dem Knochen hinweist, eine Anlagefläche 7 auf, die vorzugsweise auch an die Oberfläche des Knochens angepasst ist. Daher ist diese im Querschnitt leicht gewölbt ausgebildet.

Das Material des Grundkörpers 1 ist vorzugsweise derart ausgebildet, dass es geeignet ist, den medizinischen beziehungsweise klinischen Anforderungen gerecht zu werden.

Das Auflage- und Fixierelement 2 weist Bohrungen 5 auf, die sich bei dem hier dargestellten Ausführungsbeispiel senkrecht zur Längserstreckung der Bearbeitungslehre S erstrecken. Diese Bohrungen 5 dienen dazu, diese Bearbeitungslehre S, insbesondere das Auflage- und Fixierelement 2 an einem Knochen zu fixieren. Eine Alternative Ausbildung sieht vor, dass die Bohrungen 5 schräg angeordnet sind. Ein weiteres Ausführungsbeispiel sieht vor, dass die Borungen 5 schräg und doppelreihig ausgebildet sind, wobei die schräge Bohrungen der jeweiligen Reihe versetzt angeordnet sind. Dies bedeutet, dass eine Reihe eine positive, die weitere Reihe eine negative Winkel zur Senkrechten aufweist.

Zur Aufnahme der Adapterelemente ist an dem Anschlusselement 3 eine Ausnehmung 8 vorgesehen, die dazu dient, zumindest ein Teil der Adapterelemente aufzunehmen, so dass zumindest ein Formschluss zwischen dem Adapterelement und der übrigen Bearbeitungslehre S beziehungsweise dem Grundkörper 1 entsteht. Um auch einen Kraftschluss zwischen dem Adapterelement und der Ausnehmung 8 herzustellen, ist beispielsweise eine gefederte Druckkugel vorgesehen, die ein Klemmen des Adapterelements in der Ausnehmung 8 bewirkt.

Das freie Ende des Anschlusselements 3 weist abstehende Begrenzungselemente 10 auf. Diese Begrenzungselemente 10 dienen dazu, eine auf einer Auflagefläche 9, die ebenfalls an dem Anschlusselement 3 angeordnet ist, angelegtes Sägeblatt in seinen Bewegungen entsprechend zu begrenzen, so dass umliegendes Gewebe nicht unbeabsichtigt zerstört werden kann.

Das Anschlusselement 3 hat somit unterschiedliche Funktionen. Es dient dazu, Adapterelemente aufzunehmen, um weitere Unterstützung bezüglich einer vorgegebenen mechanischen Bearbeitung bereitzustellen. Weiterhin besteht die Funktion des Anschlusselements 3 darin, bereits eine Bearbeitungslehre S zu bilden, in dem ein Sägeblatt auf der Auflagefläche 9 angelegt wird. Die Bewegung des Sägeblattes wird durch die Begrenzungselemente 10 entsprechend eingeschränkt. Die Auflagefläche 9 ist vorzugsweise senkrecht zur Längserstreckung der Bearbeitungslehre S beziehungsweise des Grundkörpers 1 ausgebildet.

Auf der anderen Seite des Aufnahme- und Fixierelements 2 erstreckt sich das Funktionselement 4. Bei dem in den Figuren 1 bis 4 dargestellten Beispiel ist das Funktionselement 4 in einer anderen Ebene angeordnet als die, die durch das Anschlusselement 3 oder auch das Auflage- und Fixierelement 2 gebildet wird. Eine alternative Ausführungsform kann jedoch auch vorsehen, dass eine weitere Ebene oder aber auch die gleiche Ebene wie das Anschlusselement 3 beziehungsweise Auflage- und Fixierelement 2 bildet.

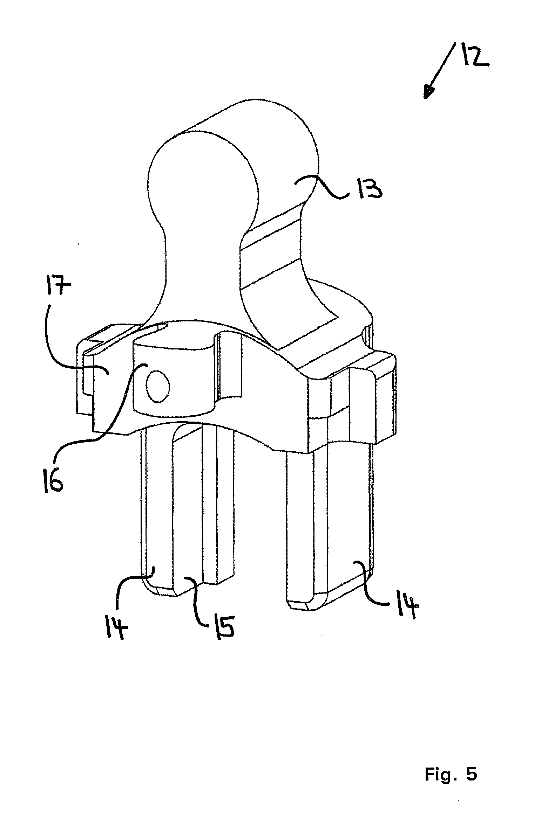

Figur 5 zeigt ein Adapterelement, das von dem Anschlusselement 3 des Grundkörpers 1 der Bearbeitungslehre S aufgenommen werden kann. Dieses Adapterelement wird auch als Ausrichtelement 12 bezeichnet. Es ist klammerartig ausgebildet und weist zwei von einem Haltegriff 13 wegweisende Schenkelelemente 14 auf. Diese Schenkelelemente 14 sind dazu geeignet, von einem später beschriebenen Werkzeug aufgenommen zu werden, da in den Schenkelelementen 14 Führungsschienen 15 angeordnet sind. Zwischen den Schenkelelementen 14 und dem Haltegriff 13 ist ein Rastelement 16 vorgesehen, das dazu geeignet ist, von der Ausnehmung 8 des Anschlusselementes 3 des Grundkörpers 1 an der Bearbeitungslehre S aufgenommen zu werden. Zusätzlich dient eine im Bereich des Rastelements 16 vorgesehene Anlagefläche 17 dazu, eine formschlüssige Anlage des Ausrichtelements 12 an dem Grundkörper 1 der Bearbeitungslehre S bereitzustellen.

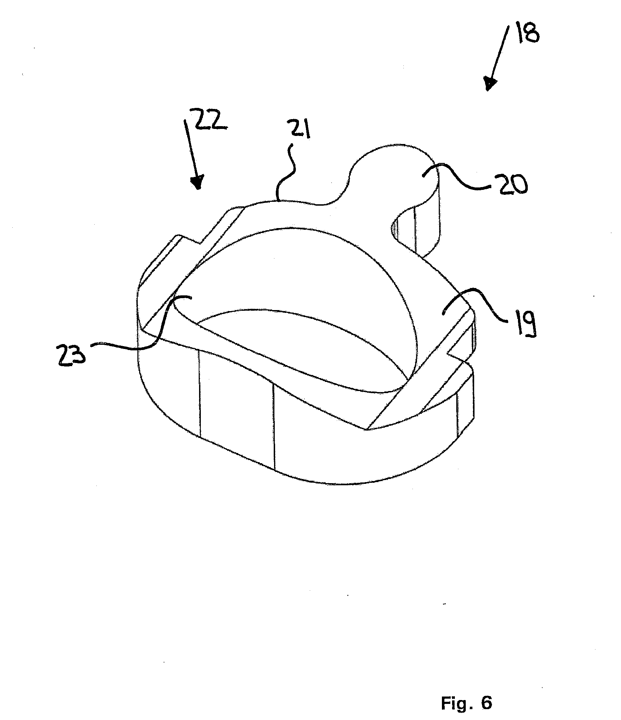

Figur 6 zeigt perspektivisch ebenfalls ein Adapterelement, das als Führungselement 18 bezeichnet wird. Das Führungselement 18 hat die Aufgabe, ein Werkzeug, beispielsweise einen Fräser oder eine Raspel während einem Bearbeitungsprozess zu führen. Dieses Führungselement 18 weist ebenfalls einen Grundkörper 19 auf, von dem ein Rastelement 20 wegweist, das wiederum dazu geeignet ist, von dem Anschlusselement 3 des Grundkörpers 1 der Bearbeitungslehre S aufgenommen zu werden. Auch eine speziell ausgebildete Anlagefläche 21 dient wie bereits beim zuvor beschriebenen Ausrichtelement 12 dazu, eine formschlüssige Verbindung mit dem Grundkörper 1 einzugehen. Ferner ist im Grundkörper 19 eine Ausnehmung 22 vorgesehen, die bei dem hier dargestellten Ausführungsbeispiel des Raspelführungselements 18 oval ausgebildet ist. Die Ausnehmung 22 weist eine innenliegende Anlagefläche 23 auf, die dazu geeignet ist, eine Führung für innerhalb der Ausnehmung 22 eingeführte Raspelwerkzeuge bereitzustellen. Die Anlagefläche 23 kann jedoch alternativ auch als "acht" ausgebildet sein, so dass das Werkzeug in Ausbildung einer "acht" entsprechend geführt wird.

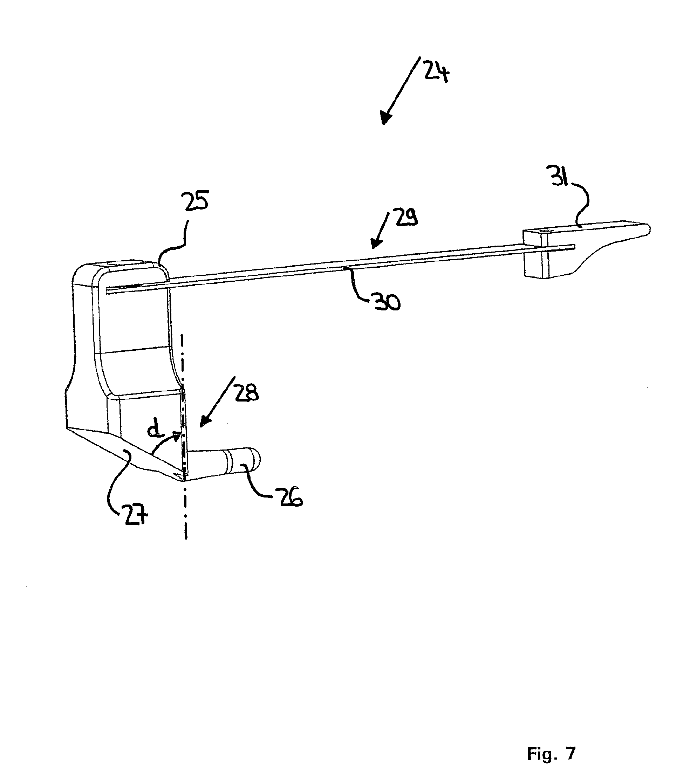

In Figur 7 ist ein weiteres Adapterelement dargestellt. Dieses Adapterelement wird als weitere Bearbeitungslehre 24 bezeichnet. Es umfasst ebenfalls einen Grundkörper 25, der als kennzeichnendes Merkmal einen Zapfen 26 aufweist. Dieser Zapfen 26 dient dazu, um die weitere Bearbeitungslehre 24 beziehungsweise deren Zapfen 26 in eine durch eine Ahle oder eine Raspel hergestellte Ausnehmung innerhalb des Knochens einzuführen. Der Zapfen 26 entspricht zumindest ungefähr dem Zapfen, der auch an einem Implantat vorgesehen ist. Als weiteres wesentliches Kennzeichen der weiteren Bearbeitungslehre 24 ist eine Anlagefläche 27 vorgesehen. Die Anlagefläche 27 ist zur Senkrechten in einem definierten Winkel α angeordnet. Da der Grundkörper 25 auf seiner zum Zapfen 26 zugewandten Seite eine Anlagefläche 28 aufweist, die mit dem Anschlusselement 3 des Grundkörpers 1 der Bearbeitungslehre S korrespondiert, kann in Bezug auf die Bearbeitungslehre S der definierte Winkel α an dem Knochen durch entsprechende Materialbearbeitung erzeugt werden. Von dem Grundkörper 1 weg erstreckt sich ein Befestigungselement 29. Dieses Befestigungselement 29 ist dadurch gekennzeichnet, dass es eine stabförmige Ausbildung 30 sowie ein an dem freien Ende der stabförmigen Ausbildung 30 angebrachten Rastelement 31 aufweist. Dieses Rastelement 31 verrastet im angebrachten Zustand der weiteren Bearbeitungslehre 24 an den Grundkörper 1 mit dem in den Figuren 1 bis 4 dargestellten Fu nktionselement 4. Dort verrastet dieses Rastelement 31 mit entsprechend vorgesehenen Rastvorrichtungen form- und kraftschlüssig.

Dieser Form- und Kraftschluss ist deswegen notwendig, da insbesondere beim Sägevorgang rüttelnde oszillierende Bewegungen entstehen. Dadurch wird vermieden, dass sich die an dem Anschlusselement 3 angebrachte weitere Bearbeitungslehre 24 ungewollt löst.

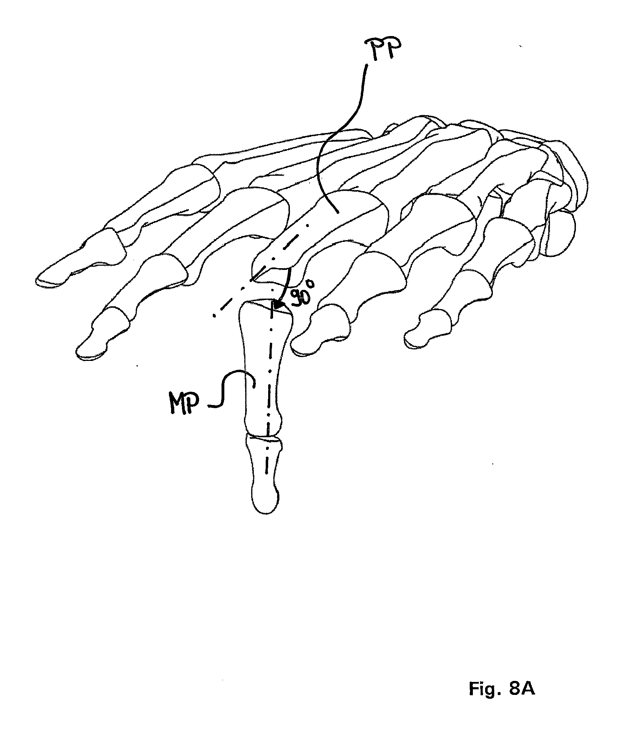

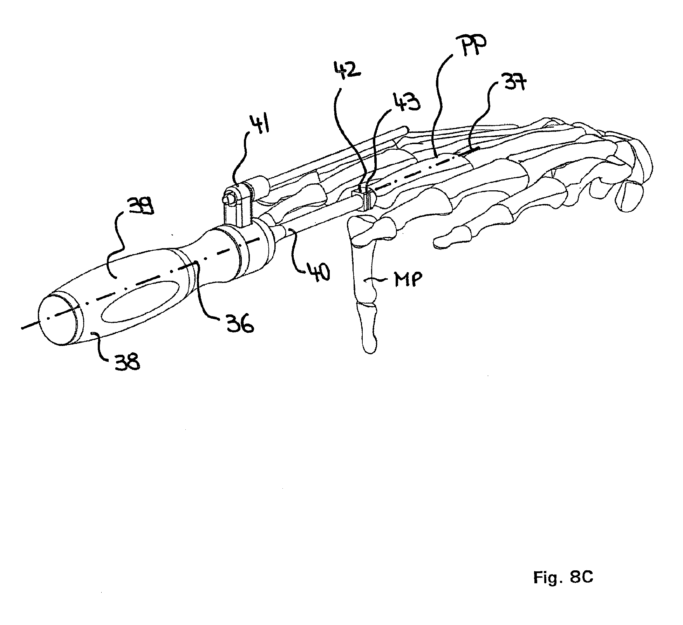

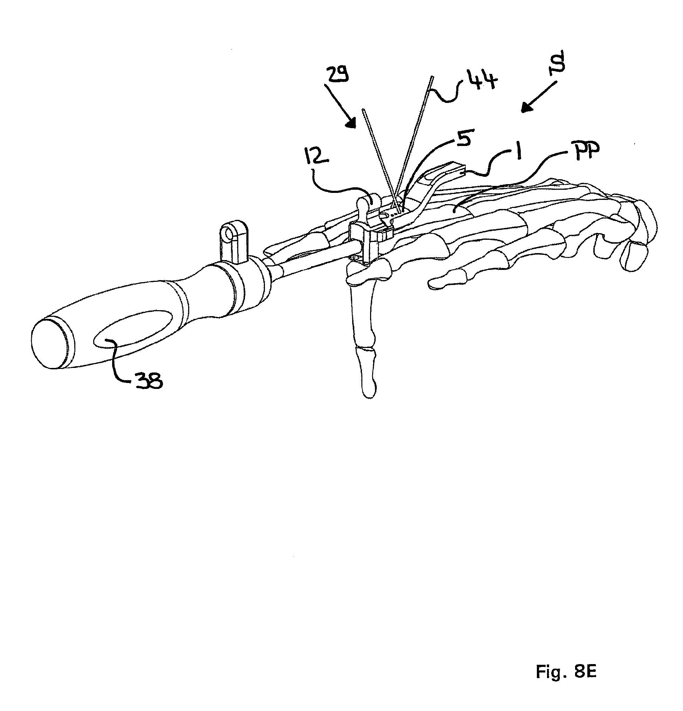

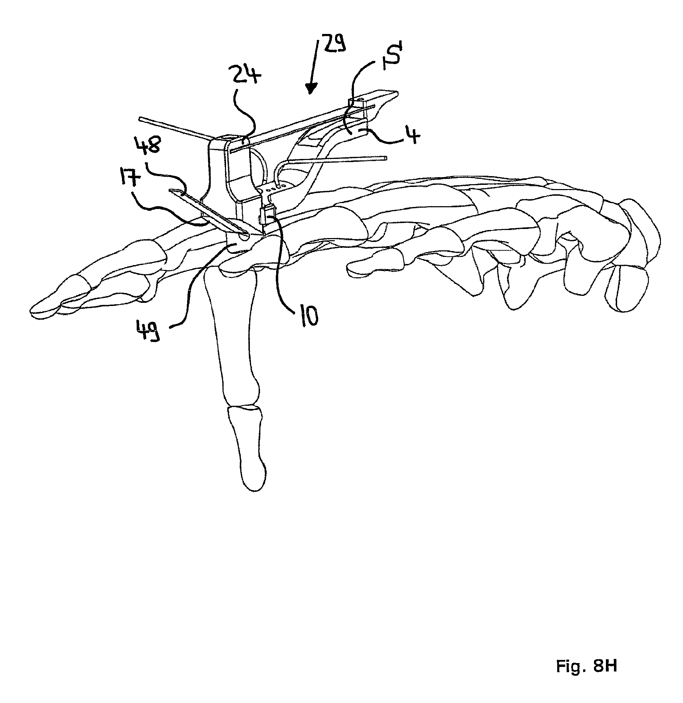

Funktionsweise der Bearbeitungslehre gemäss den Figuren 1 bis 7 In den nachstehenden Figuren 8 A bis H ist die Funktionsweise und Anwendung der Bearbeitungslehre S sowie die Darstellung des erfindungsgemässen Verfahrens zur Herstellung einer Resektionsfläche an einem freien Ende eines Knochens zum Anbringen eines Implantats dargestellt.

Figur 8A zeigt beispielhaft die Vorbereitung einer proximalen Phalanx PP zur Anbringung eines Implantats.

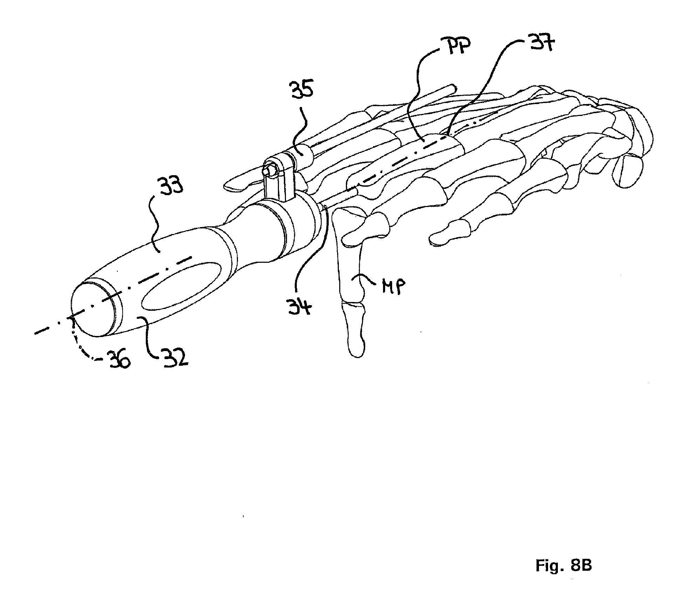

Hierzu ist es notwendig, dass die mittlere Phalanx MP nahezu in einem Winkel von 90 Grad nach unten, entsprechend zur Handflächeninnenseite in Bezug auf die proximale Phalanx PP, abgeklappt wird (Fig. 8A). Um die gewünschte Relation einer in der proximalen Phalanx PP erzeugten Aushöhlung zur zu erzeugenden Resektionsfläche herzustellen, wird gemäss Figur 8B eine Ahle 32 in die als Röhrenknochen ausgebildete proximale Phalanx PP eingeführt. Die Ahle 32 ist ein Werkzeug, das einen Griff 33 und einen von dem Griff wegweisende Ahlekörper 34 aufweist. Der Ahlekörper 34 ist zu seinem freien Ende hin spitz ausgebildet, so dass er mit entsprechendem Kraftaufwand in den Hohlraum des Röhrenknochens einführbar ist. An dem Griff 33 ist ortsfest ein Ausrichtungselement 35 vorgesehen. Mit diesem Ausrichtungselement 35 kann in Draufsicht auf die Hand die Achsparallelität von der durch die Ahle 32 gebildeten Achse 36 und die durch die proximale Phalanx PP durch deren Längserstreckung gebildete Achse 37 in Draufsicht überprüft werden. Vorzugsweise kann auch über Röntgenaufnahmen oder ähnliche Einrichtungen der Verlauf der Ahle 32 beziehungsweise des Ahlekörpers 34 kontrolliert und entsprechend korrigiert werden.

Sobald die entsprechende Aushöhlung durch die Ahle 32 hergestellt ist, wird diese entfernt, wobei die Position der mittlere Phalanx MP und der proximalen Phalanx PP beibehalten wird. In die Aushöhlung wird nun ein weiteres Werkzeug, nämlich eine Raspel 38, eingeführt. Diese Raspel 38 weist einen Griff 39 auf, von dem sich ein Raspelkörper 40 wegerstreckt. An dem Griff 39 selbst ist ortsfest, ebenfalls wie von der Ahle 32 bekannt, ein Ausrichtelement 41 zur Überprüfung der Achsparallelität von der Raspel 38 zur Achse 37 der proximalen Phalanx PP vorgesehen. Der Raspelkörper 40 weist an seinem freien Ende (welches nicht näher dargestellt ist) Raspelelemente auf, die dazu dienen, in den Hohlraum des Röhrenknochens beziehungsweise der proximalen Phalanx PP entsprechend Material abzutragen.

Je nach Ausbildung der proximalen Phalanx PP und des Hohlraumes kommen wahlweise unterschiedliche Durchmesser des Raspelkörpers 40 zum Einsatz. Alle weisen jedoch das zusätzliche Funktionselement auf, dass an dem Raspelkörper 40 ein Funktionselement 42 vorgesehen ist. Dieses Funktionselement 42 dient dazu, das Ausrichtelement 12 gemäss der Darstellung der Figur 5 aufzunehmen, in dem die Schenkelelemente 14 beziehungsweise die in den Schenkelelementen 14 angeordneten Führungsschienen 15 in Führungselemente 43 des Funktionselements 42 eingreifen. Damit kann eine lage- und formgerechte Aufnahme des Ausrichtelements 12 auf dem Auflage- und Funktionselement 2 stattfinden. Das Auflage- und Funktionselement 2 weist zusätzlich eine in Richtung proximaler Phalanx PP konisch zulaufende Form auf. Sie dient dazu, zusätzlich die Zentrierung der Raspel 38 in Bezug auf die durch die Ahle 32 ausgebildete Aushöhlung in der proximalen Phalanx PP zu unterstützen.

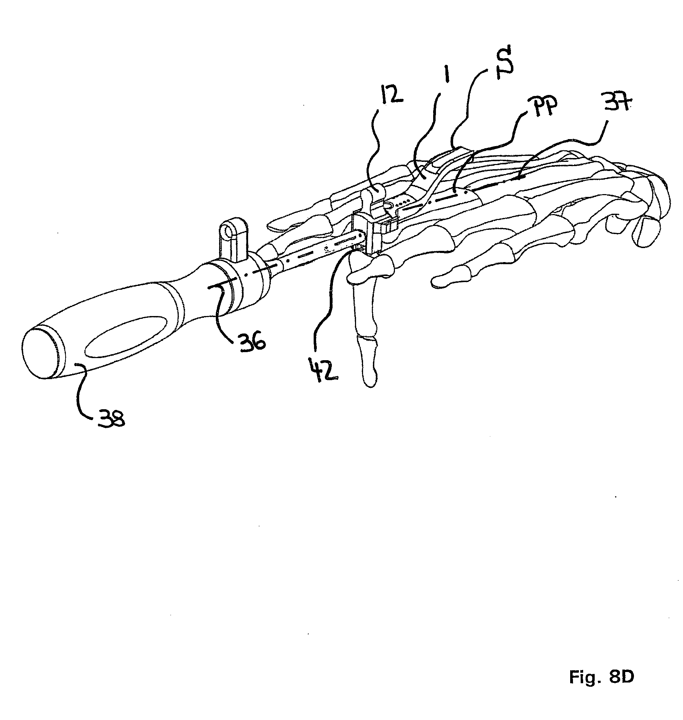

Ist die gewünschte Grösse der Aushöhlung innerhalb der proximalen Phalanx PP durch die Raspel 38 erreicht, so wird gemäss Figur 8D die Bearbeitungslehre S derart ausgebildet, dass der Grundkörper 1 der Bearbeitungslehre S mit dem Ausrichtelement 12 zusammengefügt wird und die beiden zusammengefügten Elemente dann auf das Funktionselement 42 der Raspel 38 aufgesetzt werden. Sofern das Ausrichtelement 41 störend wirkt, kann dieses auch, so wie es in Figur 8D dargestellt ist, entfernt werden.

Durch diese Ausführungsform wird gewährleistet, dass eine Achsparallelität von der Achse 37 der proximalen Phalanx PP erzielt wird. Um diese Achsparallelität weiterhin zu gewährleisten, wird in den weiteren Schritten der Grundkörper 1 an der proximalen Phalanx PP fixiert. Somit ist jedes an den Grundkörper 1 angebrachte Adapterelement ausgerichtet an der Achse 37 der proximalen Phalanx PP und kann so eine sehr genaue Bearbeitung der Resektionsfläche für die Vorbereitung und Aufnahme eines Implantats gewährleisten. Kirschner-Drähte 44, wie in Figur 8E dargestellt sind, dienen dazu, nachdem Sie durch die Bohrungen 5 des Grundkörpers 1 hindurchgeführt werden, die Bearbeitungslehre S auf der proximalen Phalanx PP zu fixieren. Je nach Beschaffenheit der proximalen Phalanx PP mit ein oder mehreren Kirschner-Drähten 44 möglich. Die entsprechenden Bohrungen 5 des Grundkörpers 1 lassen eine entsprechende Wahl zu.

Die Ahle 32 zusammen formschlüssig verbunden mit dem Ausrichtelement 12 (und dieses mit dem Grundkörper 1 der Bearbeitungslehre S), bleiben bei der Fixierung des Grundkörpers 1 auf dem Knochen unverändert.

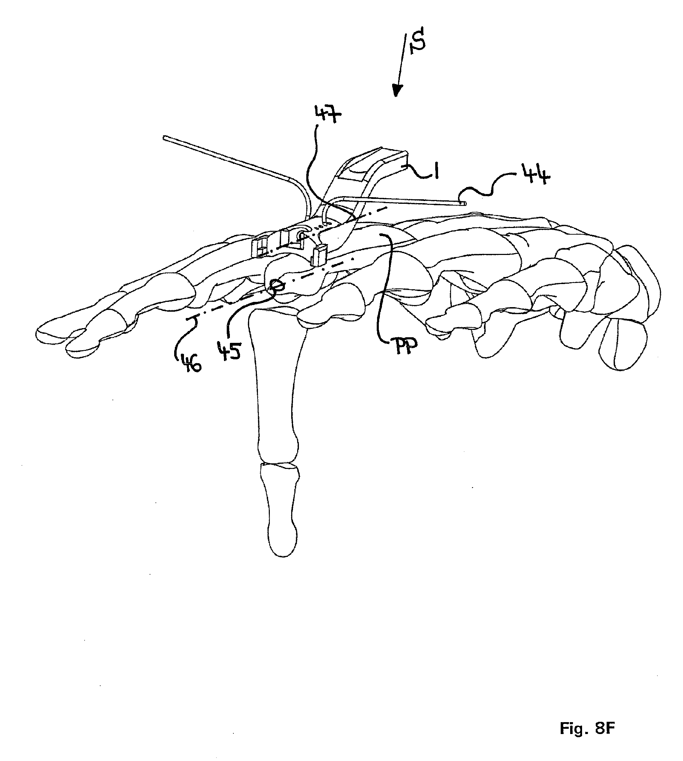

In Figur 8F ist nun die fixierte Bearbeitungslehre S mit dem Grundkörper 1 und den gebogenen Kirschner-Drähten 44 dargestellt. In der proximalen Phalanx PP ist die Aushöhlung 45 zu erkennen, die durch die Ahle 32 beziehungsweise durch die Raspel 38 erzeugt worden ist. Sie dient später dazu, einen Zapfen des Implantats aufzunehmen. Durch die Fixierung der Bearbeitungslehre S ist ein unveränderbarer Bezug zur Achse der Aushöhlung 46 und Achse des Grundkörpers 47 der Bearbeitungslehre S entstanden.

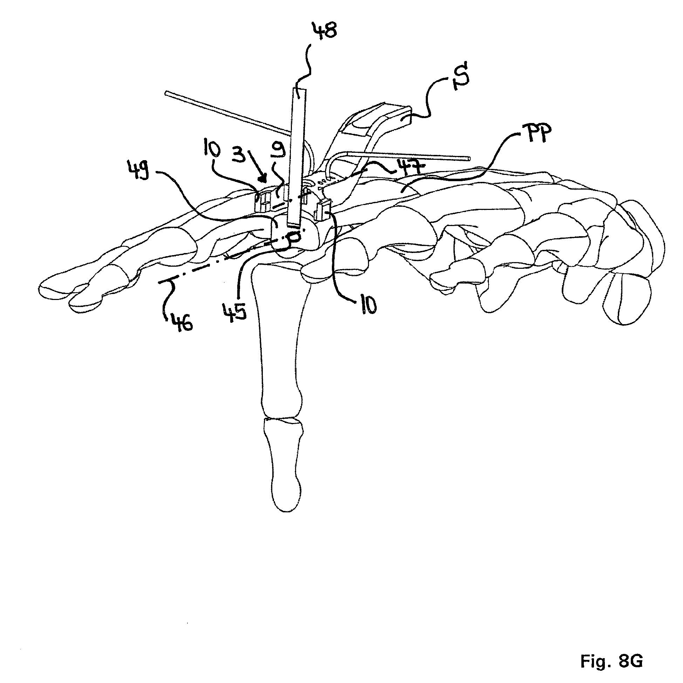

In einem weiteren Schritt, der in Figur 8G dargestellt ist, wird nun mit einer Säge, vereinfacht ist nur das Sägeblatt 48 dargestellt, durch den ersten Schritt einer Resektionsfläche 49 an der proximalen Phalanx PP hergestellt. Dies wird derart bewirkt, dass das Sägeblatt 48 entlang der Auflagefläche 9 des Anschlusselements 3 der Bearbeitungslehre S geführt wird. Die seitlich angeordneten Begrenzungselemente 10 dienen, wie bereits zuvor beschrieben, dazu, dass vermieden werden kann, dass das Sägeblatt 48 ungewollt in das benachbarte Gewebe eindringen kann. Ferner wird durch die Anlage des Sägeblattes 48 an der Auflagefläche 9 bewirkt, dass keine metallischen Späne entstehen, da die Sägeblätter in der Regel oszillierend dargestellt sind und die Sägezähne ausserhalb der Auflagefläche 9 in Einsatz mit dem Knochen gelangen. Die Aushöhlung 45 bleibt weiterhin bestehen. Die Achse der Aushöhlung 46 wird in Bezug auf die Achse 47 der Bearbeitungslehre S nicht verändert. Um nun die Resektionsfläche 49 zu vollenden, ist es notwendig, einen Teil der Resektionsfläche 49 anzuschrägen. Vorzugsweise wird senkrecht ein Winkel von 60 Grad vorgegeben. Dies geschieht derart, dass an die fixierte Bearbeitungslehre S das zuvor beschriebene und die in der Figur 7 dargestellte weitere Bearbeitungslehre 24 form- und kraftschlüssig verbunden wird. Deutlich ist zu erkennen, wie der Kraft- und Formschluss des Befestigungselements 29 mit dem Funktionselement 4 erfolgt. Die Anlagefläche 17 der weiteren Bearbeitungslehre 24 dient nun dazu, das zuvor beschriebene Sägeblatt 48 entsprechend aufzunehmen, so dass dieses flächenmässig an der Anlagefläche 17 entlang geführt werden kann. Zusätzlich sind wiederum die Begrenzungselemente 10 an der Bearbeitungslehre S wirksam, die verhindern, dass das Sägeblatt 48 in das benachbarte Gewebe eindringen kann. Damit ist die Resektionsfläche 49 vollständig hergestellt und das Implantat kann an- und eingepasst werden.

### Bezugszeichenliste

- S: Bearbeitungslehre
- MP: mittlere Phalanx
- PP: proximale Phalanx
- α: Winkel
- 1: Grundkörper
- 2: Auflage- und Fixierelement
- 3: Anschlusselement
- 4: Funktionselement
- 5: Bohrung
- 6: Unterseite
- 7: Anlagefläche
- 8: Ausnehmung
- 9: Auflagefläche
- 10: Begrenzungselemente
- 11: (nicht vergeben)
- 12: Ausrichtelement
- 13: Haltegriff
- 14: Schenkelelemente
- 15: Führungsschienen
- 16: Rastelemente
- 17: Anlagefläche
- 18: Führungselement
- 19: Grundkörper
- 20: Rastelement
- 21: Anlagefläche
- 22: Ausnehmung
- 23: Anlagefläche
- 24: Bearbeitungslehre
- 25: Grundkörper
- 26: Zapfen
- 27: Anlagefläche
- 28: Anlagefläche
- 29: Befestigungselement
- 30: stabförmige Ausbildung
- 31: Rastelement
- 32: Ahle
- 33: Griff
- 34: Ahlekörper
- 35: Ausrichtungselement
- 36: Achse
- 37: Achse proximale Phalanx
- 38: Raspel
- 39: Griff
- 40: Raspelkörper
- 41: Ausrichtelement
- 42: Funktionselement
- 43: Führungselement
- 44: Kirschner-Drähte
- 45: Aushöhlung
- 46: Achse der Aushöhlung
- 47: Achse des Grundkörpers
- 48: Sägeblatt
- 49: Resektionsfläche

## Patentansprüche

1. Bearbeitungslehre zur Herstellung einer Resektionsfläche an einem Knochen zum Anpassen eines Implantats, wobei die Bearbeitungslehre zumindest ein Auflage- und Fixierelement zum Auflegen und Befestigen an dem Knochen und eine Anlagefläche zum Führen eines Werkzeugs umfasst, **dadurch gekennzeichnet, dass** die Bearbeitungslehre (S) ein Anschlusselement (3) umfasst, das geeignet ist, eine formschlüssige Verbindung zu einem Adapterelement bereitzustellen, das wiederum mittelbar oder unmittelbar an ein Werkzeug zur Herstellung einer Aushöhlung (45) in dem Knochen geeignet ist.

2. Bearbeitungslehre nach Anspruch 1, **dadurch gekennzeichnet, dass** an dem Anschlusselement (3) ein lösbares Adapterelement in der Ausführung eines Ausrichtelements (12) vorgesehen ist, wobei das Ausrichtelement (12) auf ein Adapterelement eines Werkzeugs zur Herstellung einer Aushöhlung (45) vorgesehen ist.

3. Bearbeitungslehre nach Anspruch 1 oder 2, **dadurch gekennzeichnet, dass** im Bereich des Anschlusselements (3) eine Anlagefläche (9) zur Führung eines Werkzeugs in Form eines Sägeblattes (48) vorgesehen ist, wobei sich diese Anlagefläche (9) senkrecht zur Längserstreckung der Bearbeitungslehre (S) erstreckt.

4. Bearbeitungslehre nach mindestens einem der vorhergehenden Ansprüche, **dadurch gekennzeichnet, dass** an dem Anschlusselement (3) eine weitere Bearbeitungslehre (24) anbringbar ist, die eine Anlagefläche (17) zur Aufnahme eines Sägeblattes (48) bereitstellt.

5. Bearbeitungslehre nach mindestens einem der vorhergehenden Ansprüche, **dadurch gekennzeichnet, dass** die Bearbeitungslehre (S) Bohrungen (5) umfasst, die mit Befestigungselementen (29) zur Befestigung der Bearbeitungslehre (S) an dem Knochen zusammenwirken.

6. Bearbeitungslehre nach Anspruch 5, **dadurch gekennzeichnet, dass** das Befestigungselement (29) zumindest ein Kirschner-Draht (44) ist.
